(19)

Europäisches Patentamt

European Patent Office

Office européen des brevets

(11) **EP 2 407 782 A1**

(12) **EUROPEAN PATENT APPLICATION**

(43) Date of publication:
**18.01.2012 Bulletin 2012/03**

(51) Int Cl.:
***G01N 33/50*** *(2006.01)*

(21) Application number: **11166862.0**

(22) Date of filing: **11.07.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.07.2006  US 830296 P**

(62) Document number(s) of the earlier application(s) in accordance with Art. 76 EPC:
**10006534.1 / 2 287 606**
**07766899.4 / 2 041 564**

(27) Previously filed application:
**11.07.2007 EP 10006534**

(71) Applicant: **Procognia (Israel) Ltd.**
**Ashdod 77610 (IL)**

(72) Inventors:
• **Rosenfeld, Rakefet**
**71908, Maccabim (IL)**
• **Samokovlisky, Albena**
**77644, Ashdod (IL)**

• **Maya, Ruth**
**73142, Shoham (IL)**
• **Yakir, Yeshayahu**
**75258, Rishon LeZion (IL)**
• **Landstein, Dorit**
**60946, Moshav Bitzaron (IL)**
• **Zalle, Noa**
**89550, Mahane Adi (IL)**
• **Aloni, Ronny**
**34324, Haifa (IL)**

(74) Representative: **Virdee-Crofts, Kulwinder Kaur et al**
**Fisher Weiler Group**
**Portland House**
**Bressenden Place**
**London, SW1E 5RS (GB)**

Remarks:
This application was filed on 20-05-2011 as a divisional application to the application mentioned under INID code 62.

(54) **Method and assay for glycosylation pattern detection related to cell state**

(57) A method and assay for characterizing populations of cells according to their glycosylation pattern, particularly for distinguishing between cell populations, through binding of at least one saccharide binding agent. In preferred embodiments the present invention able to determine the state of stem cell (i.e. differentiated or undifferentiated) and/or the state of a cancer cell, for example with regard to malignancy. Preferably the present invention is also able to determine whether a patient is likely to respond to a drug according to the glycosylation pattern of a sample of cancer cells taken from the patient (or alternatively examined while in the patient, as described in greater detail below). Also optionally, it may be used to analyze a cell population before and after treatment with a drug for example.

EP 2 407 782 A1

**Description**

FIELD OF THE INVENTION

The present invention relates to a method and assay for detecting glycosylation patterns of cells, and in particular, to such a method and assay which enable the state of a cell to be determined according to the detected glycosylation pattern.

BACKGROUND OF THE INVENTION

Oligosaccharides and polysaccharides are polymers that consist of monosaccharide (sugar) units, connected to each other via glycosidic bonds. These polymers have a structure that can be described in terms of the linear sequence of the monosaccharide subunits, which is known as the two-dimensional structure of the polysaccharide. Polysaccharides can also be described in terms of the structures formed in three dimensions by their component monosaccharide subunits.

The saccharide chain has, like a chain of DNA or protein, two dissimilar ends. In the case of saccharide chains, these are the reducing end (corresponding to the aldehyde group of the linear sugar molecule) and the non-reducing end. Unlike proteins and DNA, however, polysaccharides are generally branched, with essentially each of the sugar units in the polysaccharide serving as an optional branching point.

There are a number of proteins that bind to saccharides. Many of these proteins bind specifically to a certain short mono or disaccharide sequence. Lectins are a broad family of proteins that bind saccharides. A large number of plant lectins have been characterized and are used in research. Many mammalian lectins have also been characterized. Antibodies are proteins that specifically recognize certain molecular structures. Antibodies may also recognize saccharide structures, as do lectins. Glycosidases are enzymes that cleave glycosidic bonds within the saccharide chain. Also glycosidases may recognize certain oligosaccharide sequences specifically. Glycosyltransferases are enzymes that transfer a sugar unit to acceptor molecules. In vivo, these acceptor molecules are the growing glycan structures.

The structural determination of polysaccharides is of fundamental importance for the development of glycobiology. Research in glycobiology relates to subjects as diverse as the bacterial cell walls, blood glycans, to growth factor and cell surface receptor structures involved in viral disease, such as HIV infection, autoimmune diseases such as insulin-dependent diabetes and rheumatoid arthritis, and abnormal cell growth as it occurs in cancer.

The importance of glycomolecules is highlighted by the discovery of penicillin, an inhibitor of glycomolecule synthesis in the bacterial cell-wall and possibly the most successful antibiotic discovered to date.

Another example is the medical use of heparin, a glycosaminoglycan that inhibits blood clotting and is today widely used in medicine. Further examples of medically-important glycomolecules include: glycosaminoglycans (GAGs), heparan sulphate, monoclonal antibodies, cytokines (e.g. IL-8, TNF, and the blockbuster EPO), chemokines (e.g. acidic fibroblast growth factor) and various growth factors. The aforementioned cytokines, chemokines and growth factors are also capable of binding to GAGs and other polysaccharides, and therefore may be also be considered to be lectins.

The structural complexity of polysaccharides has hindered their analysis. For example, saccharides are believed to be synthesized through a template-independent mechanism. In the absence of structural information, the researcher must therefore assume that the building units are selected from any of the saccharide units known today. In addition, these units may have been modified, during synthesis, e. g., by the addition of sulfate groups. Without the ability to measure such carbohydrate structural information, the researcher cannot determine the true, correct glycosylation pattern for populations of cells, for example in a tissue. In addition, these units may have been modified, e.g. by the addition of sulfate groups, during synthesis, such that merely understanding which types of saccharides may have been added does not provide a complete picture.

Furthermore, the connections between saccharide units are multifold. A saccharide may be connected to any of the C1, C2, C3, C4, or C6 atoms if the sugar unit to which it is connected is a hexose. Moreover, the connection to the Cl atom may be in either alpha or beta configuration. In addition, the difference in structure between many sugars is minute, as a sugar unit may differ from another merely by the position of the hydroxyl groups (epimers).

In vivo, glycosylation is tissue dependant and can vary significantly with cell state. In vitro, glycosylation strongly depends on growth conditions: the type of cell, nutrient concentrations, pH, cell density, and age can affect the glycosylation patterns of glycoproteins. The number of glycoforms and their relative abundance within a cell are affected by the intrinsic structural properties of the individual protein, as well as the repertoire of glycosylation enzymes available (including their type, concentration, kinetic characteristics, compartmentalization). This repertoire has been shown to change upon changes in cell state (e.g. oncogenic transformation).

SUMMARY OF THE INVENTION

There is a need for a method and assay for detecting glycosylation patterns, and their relationship to cell state.

The present invention overcomes at least some of the deficiencies of the background art by providing such a method and assay, which in preferred embodiments are able to determine the state of a cell according to the glycosylation pattern for a plurality of different but correlated glycomarkers. Optionally and preferably, preferred embodiments of the present invention are able to determine the glycosylation pattern of cells in at least two different cell populations, and to correlate the glycosylation pattern with one or more characteristics of each cell population, for example according to the state of the cells.

According to a preferred embodiment, the present invention provides a method of detecting the state of a cell, the method comprising contacting at least a portion of a cell with at least one saccharide-binding agent, determining binding of the saccharide-binding agent to the cell, determining the glycosylation pattern of the cell according to the binding of the saccharide-binding agent to the cell, and correlating the glycosylation pattern to the state of the cell.

According to some embodiments, at least two saccharide-binding agents are used in a single assay, preferably using whole cells (which may optionally be fixed), and/or non-whole cell material. Such non-whole cell material may optionally include a material selected from the group consisting of membrane protein extracts, homogenized cells, crude membrane mixture, crude cell mixture and/or any non-whole material derived from adding detergent and/or performing solubilization and/or extraction to cells. More preferably, the non-whole cell material is a crude cell mixture rather than a highly purified protein or group of proteins.

According to other embodiments, at least five saccharide-binding agents are used, preferably using whole cells (which may optionally be fixed), and/or non-whole cell material as described above.

For example preferred embodiments of the present invention are able to determine the state of a stem cell (i.e. differentiated or undifferentiated) and/or the state of a cancer cell, for example with regard to malignancy. Preferably the present invention is also able to determine whether a patient is likely to respond to a drug according to the glycosylation pattern of a sample of cancer cells taken from the patient (or alternatively examined while in the patient).

As described in greater detail below, according to preferred embodiments of the present invention, the method and assay of the present invention are preferably performed in vitro, on a sample of cells and/or cell material.

The sample is preferably contacted with a glycomolecule detecting agent as described in greater detail below, such that at least a portion of the glycomolecules present in the sample are detected. A glycosylation fingerprint is then preferably determined for the sample, which is then preferably correlated with a state of a cell. Optionally such a state is related to a state of differentiation, as for example for a stem cell; alternatively or additionally, and optionally, such a state is related to a predicted response of the individual from which the sample was taken to a therapy, such as for chemotherapy for cancer for example.

Optionally and preferably, such a correlation is performed according to a comparison, such that if a glycosylation pattern matches a first category, then the sample correlates with a first cell state; alternatively if the glycosylation pattern matches a second category, then the sample correlates with a second cell state. More preferably, such a correlation may optionally feature a plurality of different categories relating to a plurality of different states, which most preferably fall along a continuum of cell functionality and/or behavior. The cell state is determined using the minimum number of data inputs required to differentiate between the different states. The first and second category may be, for example a cancerous and a non-cancerous state, or a differentiated and undifferentiated state.

According to some embodiments, the present invention therefore also relates to diagnostic assays for disease detection optionally and preferably in a biological sample taken from a subject (patient), which is more preferably some type of body fluid or secretion, including but not limited to seminal plasma, blood, serum, urine, prostatic fluid, seminal fluid, semen, the external secretions of the skin, respiratory, intestinal, and genitourinary tracts, tears, cerebrospinal fluid, sputum, saliva, milk, peritoneal fluid, pleural fluid, cyst fluid, broncho alveolar lavage, lavage of the reproductive system and/or lavage of any other part of the body or system in the body, and stool or a tissue sample. The term may also optionally encompass samples of in vivo cell culture constituents. Preferably, the sample contains cells, either in the form of whole cells or as broken cell components.

The biological sample is optionally and preferably analyzed for the presence of one or more markers, which may optionally comprise a glycoprotein or a polysaccharide. These markers may be specifically released to the bloodstream under conditions of a particular disease, and/or are otherwise expressed at a much higher level and/or specifically expressed in tissue or cells afflicted with or demonstrating the disease. The measurement of these markers, alone or in combination, in patient samples provides information that the diagnostician can correlate with a probable diagnosis of a particular disease and/or a condition that is indicative of a higher risk for a particular disease.

According to some embodiments, the present invention therefore also relates to diagnostic assays for marker-detectable disease and/or an indicative condition, and methods of use of such markers for detection of marker- detectable disease and/or an indicative condition, optionally and preferably in a sample taken from a subject (patient) as described above.

Unless otherwise defined, all technical and scientific terms used herein have the same meaning as commonly understood by one of ordinary skill in the art to which this invention belongs. Although methods and materials similar or equivalent to those described herein can be used in the practice or testing of the invention, suitable methods and materials are described below. All publications, patent applications, patents, and other references mentioned herein are incorporated by reference in their entirety. These include, but are not limited to, WO 00/68688 and WO 01/84147 (US20060194269, US20070092915, US7056678 and US7132251), WO 02/37106 (US20040132131), and WO 02/44714 (US7079955 and US20040153252). In the case of conflict, the present Specification, including definitions, will control. In addition, the materials, methods, and examples are illustrative only and not intended to be limiting.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1 shows the differentiation of 3T3L1 cells to adipocytes. Differentiation is initiated 48 hours after plating, when the cells are still sub-confluent. After 7 days, fat droplets appear in the cell body. By day 10, at least 80% of the cells are differentiated.

FIG. 2 shows changes in glycosylation of membrane proteins of 3T3L1 cells upon differentiation to adipocytes.

FIG. 3 shows changes in glycosylation of membrane proteins upon treatment of PC12 cells with BFA.

FIG. 4 shows changes in glycosylation of membrane proteins of 3T3L1 cells upon treatment with DMJ.

DESCRIPTION OF THE PREFERRED EMBODIMENTS

The present invention provides a method and assay for characterizing populations of cells according to their glycosylation pattern, preferably for distinguishing between cell populations. In preferred embodiments the present invention is able to determine the state of a stem cell (i.e. differentiated or undifferentiated) and/or the state of a cancer cell, for example with regard to malignancy. Hence, the method may be used to determine whether a cell is cancerous or non-cancerous, and, if cancerous, whether the cancer is benign or malignant.

Optionally, the state of the cell may be correlated to the glycosylation pattern by comparison to a known glycosylation pattern. Further optionally the glycosylation pattern may be computationally analyzed.

Preferably the present invention is also able to determine whether a patient is likely to respond to a drug. For example, the predicted response to chemotherapy may be determined according to the glycosylation pattern of a sample of cancer cells taken from the patient (or alternatively examined while in the patient, as described in greater detail below).

According to a preferred embodiment, the present invention provides a method of detecting the state of a cell, the method comprising contacting at least a portion of a cell with at least one saccharide-binding agent, determining binding of the saccharide-binding agent to the cell, determining the glycosylation pattern of the cell according to the binding of the saccharide-binding agent to the cell, and correlating the glycosylation pattern to the state of the cell.

According to preferred embodiments, the method and assay of the present invention may optionally be used to compare a plurality of biologically comparable systems through the analysis of the glycosylation pattern, preferably of a population of cells. The biological system may optionally represent any cell type physically or chemically treated to induce a cellular response, or a primitive cell induced to differentiate, a cell before and after oncogenic transduction or any other manipulation of a certain cell type.

According to preferred embodiments of the present invention, an assay for detecting a glycosylation pattern of a cell may optionally and preferably be performed according to US Patent No. 7,056,678, owned in common with the present application, hereby incorporated by reference as if fully set forth herein, which describes methods and assays for detecting glycosylation of a cell. For example, this patent describes a method for the structural analysis of a saccharide, comprising: providing on a surface a plurality of essentially sequence-specific and/or site-specific binding agents; contacting the surface with a mixture of saccharides to be analyzed, for example an extract of glycomolecules from specific compartments of cells or tissue washing or otherwise removing unbound saccharide or saccharide fragments; adding to the surface obtained previously an essentially sequence- and/or site-specific marker, or a mixture of essentially sequence-and/or site-specific markers; acquiring one or more images of the markers that are bound to the surface; and deriving information related to the identity of the saccharide being analyzed from the image.

The surface on which the binding agents are provided may comprise, for example, a bead or an array.

Binding of the saccharide-binding markers may optionally be detected by acquiring images of the markers, and generating a map of recognition sites of the polysaccharide being analyzed, to derive partial sequence information relating to the polysaccharide.

The markers may optionally comprise chromogenic binding agents, such that images are provided which are colors that develop on the surface of the substrate. Alternatively, the markers may be labeled binding agents, such that images of the markers are provided according to a signal from the label. Images may be acquired, for example, by the use of optical filters, or by photographing and/or digitizing the images.

Additional methods and assays for determining a glycosylation pattern or "fingerprint" for a sample, such as for a cell for example, are also disclosed in US Patent Application No. 20050186645, also owned in common with the present application, which is hereby incorporated by reference as if fully set forth herein. This application describes a method for obtaining information about the carbohydrate content of a glycomolecule by adding a glycomolecule to a substrate to which is attached one or more saccharide-binding agents (also referred to herein as first saccharide-binding agents). The first saccharide-binding agents that have bound the glycomolecule are identified, and the resulting binding information is used to generate a fingerprint of the glycomolecule.

The essentially sequence- and/or site-specific binding agents of the present invention may comprise, for example, lectins (such as colored lectins, fluorescent lectins, biotin labeled lectins) or antibodies (such as fluorescent antibodies, biotin-labeled antibodies, or enzyme-labeled antibodies). The method or assay may be performed using at least five lectins, such as, for example, 5, 10, 15, 20, 25, 30, 35, 40, 45 or 50 lectins, although optionally any number of lectins may be used, for example from about 5 lectins to about 100 or more lectins.

For example, the method may optionally be performed with a set of 20-30 lectins printed on a membrane-coated glass slide in replicates of 4-8, or alternatively in a range of concentrations that provide a dose-response for each printed lectin. A sample of intact glycoprotein is applied to the array, and its binding pattern is detected by either direct labeling of the glycoprotein using any fluorophore, or by using a fluorophore-labeled probe that is directed at either the protein moiety-an antibody for example, or a carbohydrate moiety--a lectin. The resulting fingerprints are highly characteristic of the glycosylation pattern of the sample. The large number of lectins, each with its specific recognition pattern, ensures high sensitivity of the fingerprint to changes in the glycosylation pattern. Many fluorescent labels such as FITC, Rhodamine, Cy3, Cy5, or any of the Alexa dyes can be used. These fluorescent labels and dye labels are collectively termed herein "chromogenic labels". In addition, labeling can be effected using biotin-avidin systems known in the art and/or with any other suitable type of label. Glycomolecules may optionally be modified before being analyzed as described above.

The method and assay of the present invention may optionally be carried out on whole cells. Alternatively, the method and assay may be carried out on a cell preparation (non-whole cell material), such as, for example, a membrane protein extract, a homogenized cell, or a crude membrane mixture.

In embodiments which comprise the use of a whole cell, the cell is preferably first fixed. For example, the cells may be fixed in suspension of RPMI culture medium by adding 1% glutaraldehyde in Sorenson's buffer, pH 7.3 (Tousimis Research Corp., Rockville, Md), and washing in Sorenson's buffer after 24-48 hours (as described for example in Sanders et al, A high-yield technique for preparing cells fixed in suspension for scanning electron microscopy, The Journal of Cell Biology, Volume 67, 1975, pages 476 480).

Alternatively, cells may be fixed by immersing in PBS / 3.7 % formaldehyde for 60 minutes at ambient temperature, after which the cells are washed in distilled water (as described for example in Nimrichter et al, Intact cell adhesion to glycan microarrays, Glycobiology, vol. 14, no. 2; pp. 197-203, 2004).

Of course any type of cell fixation process may optionally be performed which permits detection of binding of saccharide-binding agents to the cells.

The method of the present invention may optionally and preferably be performed in vitro.

The method and assay of the present invention may optionally and preferably be carried out using the Qproteome Glycoprofiling Kit (Qiagen USA). Lectins used in such kits have been chosen by analysis of a set of over 80 lectins, using a large dataset of carefully chosen, well-characterized glycoproteins, and a large set of enzymatically synthesized glycovariants of these proteins. The lectins on the array are grouped according to their monosaccharide specificities, in cases where possible; lectins in the group that is denoted "complex" do not bind monosaccharides, but bind complex N-linked glycans. The groups and differences between lectins within each group are detailed below.

**Complex**

The lectins in this group recognize branching at either of the two α-mannose residues of the tri-mannosyl core of complex N-linked complex glycans. Some of the lectins of this group are sensitive to different antennae termini as they bind large parts of the glycan structure. The lectins denoted Complex(1) and Complex(4) have a preference for 2,6-branched structures; lectin Complex(3) has a preference for 2,4-branched structures, and lectin Complex(2) recognizes with similar affinity both structures.

**GlcNAc**

The lectins in this group bind N-acetylglucosamine (GlcNAc) and its β4-linked oligomers with an affinity that increases with chain length of the latter. The carbohydrate-specificity of both lectins in this group do not differ, yet differences in their binding patterns are observed and probably stem from the non-carbohydrate portion of the samples.

**Glc/Man**

This group of lectins is a subgroup of the mannose binding lectins (see below), and are denoted Glc/Man binding lectins since they bind, in addition to mannose, also glucose. All of the lectins in this group bind to bi-antennary complex N-lined glycans with high affinity. In comparison to their affinity for bi-antennary structures, lectins Glc\Man(1) and (2) bind high mannose glycans with lower affinity, whereas lectin Glc\Man(3) will bind high mannose glycans with higher affinity.

**Mannose**

This group consists of lectins that bind specifically to mannose. These lectins will bind high mannose structures and, with lower affinity, will recognize the core mannose of bi-antennary complex structures.

**Terminal GlcNAc**

This lectin specifically recognizes terminal GlcNAc residues.

**Alpha Gal**

These lectins bind terminal α-galactose (a-Gal). Lectin Alpha-Gal(1) binds both α-galactose and α-GalNAc (α-N-acetylgalactosamine) and may bind to both N and O-linked glycans. Lectin Alpha-Gal(3) binds mainly the Galili antigen (Galal-3Gal) found on N-linked antennae.

**Beta Gal**

These lectins specifically bind terminal (non-sialylated) β-galactose residues.

**Gal/GalNAc**

These lectins are specific for terminal galactose and N-acetyl-galactoseamine residues. The different lectins within this group differ in their relative affinities for galactose and N-acetyl-galactoseamine.
Lectins (2) and (5) from this group bind almost exclusively Gal; lectins (1), (3) and (4) bind almost exclusively GalNAc. The relative affinities for GalNAc / Gal for the remaining lectins in the group are ranked: (8)> (7)> (6).

**Fucose**

Lectins from this group bind fucose residues in various linkages.
Lectin Fucose(6) binds preferentially to 1-2-linked fucose; Lectin Fucose(8) binds preferentially to 1-3 and 1-6 lined fucose; Lectins Fucose(12) and (13) bind preferentially to Fucl-4GlcNAc (Lewis A antigens).
These lectins generally do not bind the core fucose ofN-linked oligosaccharides on intact glycoproteins due to steric hindrance.

**Sialic acid**

The sialic acid lectins react with charged sialic acid residues. A secondary specificity for other acidic groups (such as sulfation) may also be observed for members of this group. Lectin Sialic Acid(1) recognized mainly 2-3-linked sialic acid; Lectin Sialic Acid(4) recognizes mainly 2-6-linked sialic acid.
The fingerprint itself provides valuable data for sample analysis. It is particularly useful for comparative analysis of several samples, to show differences in glycosylation.
Computational methods for analyzing the resultant glycosylation fingerprint data and for mapping glycosylation pattern(s) in the sample are disclosed for example in US Patent Application No. 20040153252, also owned in common with the present application, which is hereby incorporated by reference as if fully set forth herein. This application describes a method for computationally analyzing data from binding of a saccharide binding agent to a glycomolecule in the sample, such as lectin binding data for example, optionally with other types of binding data, to map the glycosylation patterns. A more detailed description of exemplary computational methods is provided below with regard to Example 9.

It should be understood that these examples for methods and assays for detecting glycosylation patterns in a sample, such as a cell for example, are provided for the purposes of discussion only and are not intended to be limiting in any way, as any other suitable method and/or assay could optionally be used with the present invention.

The principles and operation of the present invention may be better understood with reference to the drawings and the accompanying description, as well as the following examples.

Example 1

Methods for Identifying Global Glycosylation Chances in Cells

This Example relates to illustrative methods and kits for identifying changes in glycosylation pattern in cells, preferably global glycosylation changes. These are intended as examples only and are not meant to be limiting in any way.

The Qglycome cell profiling Kit can be used as a first-line tool for the identification and gross characterization of global glycosylation changes that occur upon biological changes. The kit is intended for analyzing global changes in glycosylation patterns in cell membrane protein glycosylation of cultured mammalian cells. It should be noted that the description of the kit and methods of use thereof provided herein is given in the present tense, as for some of the prospective examples below, these methods are to be performed; however for other examples given below, the methods were performed and actual data obtained. Whether the examples are to be performed or already have been performed is indicated with regard to each example below.

The analysis is performed in a comparative manner between two samples, one being the reference and the other the test sample. Examples for such comparisons can be cells before and after differentiation, and cells at time 0 in comparison to various time points following biological/chemical/ physical stimuli.

Lectins with differing, well-characterized glycan-binding specificities are spotted on the surface of an array. The array is probed with a biotinylated membrane glycoprotein mixture and washed to reduce background. Bound biotinylated glycoproteins are visualized with Cy®3-labeled streptavidin using a microarray scanner. Following scanning, the resulting images are analyzed using Qiagen GlycoAnalyzer Software.

Each lectin is printed on the slides at seven concentrations. The analysis provides a lectin fingerprint for each sample: a histogram in which each bar represents the slope of the dose-response curve observed for each lectin. The lectins in the fingerprint are grouped by their specificity, and presented in a group-number format; the group-number combination is constant for each lectin and does not change. All fingerprints in an experiment are preferably normalized to the fingerprint of the reference sample. Microarray technologies generally require normalization between slides to adjust for variations that arise from technology, rather than from biological differences. The normalization in Qproteome Glycoprofiling Kits is preferably based on a robust-regression algorithm (using MM-estimators; please see Example 9 for a description).

The kit contains 2 Extraction Buffers, which enable the sequential enrichment of proteins associated with the cytosol (CE1 buffer) and membranes (CE2 buffer) from cultured cells. This kit is preferably used for glycoanalysis of the membrane proteins.

**Cell fractionation procedure**

Extraction Buffer CE1 is added to cells and selectively disrupts the plasma membrane without solubilizing it, resulting in the release of cytosolic proteins. Plasma membranes and organelles, such as nuclei, mitochondria, and the endoplasmic reticulum (ER), remain intact and are collected by centrifugation.

The pellet from the first step is resuspended in Extraction Buffer CE2, which solubilizes all cellular membranes with the exception of the nuclear membrane. An additional centrifugation precipitates nuclei and cytoskeleton, leaving all extracted membrane proteins in the supernatant.

**Labeling membrane proteins with biotin**

The Qglyco profiling kit offers two labeling protocols:

- Labeling of total membrane protein extract with Sulfo-NHS-Biotin (the entire membrane fraction will be labeled).
- Labeling of cell surface proteins with the Sulfo-NHS-Biotin. Using this protocol, only proteins on the external surface of the cell membrane will be labeled, provided the cells are intact. The non-permeable variant of NHS-Biotin (Sulfo-NHS-biotin), enables labeling of cell surface proteins, and will not label internal proteins if the cells are intact. Higher sensitivity may be achieved using surface labeling, since glycoproteins in the Golgi and ER - which contain high levels of high mannose glycans - will not be labeled. If no significant changes in global pattern glycosylation are observed in experiments using total extract labeling, the surface labeling protocol is performed.

**Protocol: Cell Fractionation and Biotin Labeling of Total Membrane Protein Extract**

This protocol labels the entire membrane fraction, and is suitable for the processing of $5 \times 10^6$ cells in 1ml of fractionation buffer.

Extraction Buffers CE1 and CE2 are thawed, then mixed well by vortexing and placed on ice. 1 ml of each buffer is transferred to a separate pre-labeled tube and 10 $\mu$l Protease Inhibitor Solution (100x) is added.

Adherent cells are washed with 5ml PBS. PBS is then aspirated. 1ml of 2mM EDTA in PBS is added and dispersed equally over the entire dish. Once cells begin detaching, cells are collected with a scraper and transferred with a 1ml pipette into a 15 ml tube. Cells are counted, then centrifuged at 500 x g at 4°C for 10 min. The cell pellet is resuspended in 2 ml ice cold PBS, and transfer on ice. The centrifugation step is repeated and cells counted again. Fractionation is then performed as described below.

Non-adherent cells are transferred from the flask to a 15 ml or 50 ml tube. Cells are pelleted by centrifugation at 500 x g and resuspended in 2 ml ice-cold PBS. Cells are then centrifuged at 500 x g at 4°C for 10 min, the cell pellet resuspended in 2 ml ice cold PBS, and transferred on ice. The second centrifugation step is repeated, and the cells counted. Fractionation is then performed, as described below.

Fractionation

A cell suspension containing $5 \times 10^6$ cells is transferred into a microcentrifuge tube and centrifuged at 500 x g for 10 min at 4°C. The supernatant is carefully removed and discarded.

The cell pellet is resuspended in 1 ml ice-cold Extraction Buffer CE1 to which Protease Inhibitor Solution has been added, and incubated for 10 min at 4°C on an end-over-end shaker. The lysate is centrifuged at 1000 x g for 10 min at 4°C, and the supernatant (fraction 1) is carefully transferred into a fresh microcentrifuge tube, which is then stored on ice. This fraction primarily contains cytosolic proteins.

The pellet is resuspended in 1 ml ice-cold Extraction Buffer CE2, to which Protease Inhibitor Solution has been. The suspension is incubated for 30 min at 4°C on an end-over-end shaker. The suspension is centrifuged at 6000 x g for 10 min at 4°C, then the supernatant (membrane protein fraction) is carefully transferred into a fresh microcentrifuge tube and stored on ice.

This fraction primarily contains membrane proteins. This fraction can be stored at -20°C for up to three months and at -70°C for longer periods. Repeated freeze-thaw cycles are avoided. After thawing, the sample is centrifuged at 6,000 x g for 15 seconds. Before labeling, the protein concentration is determined using the BCA Protein Quantification Kit.

The minimum protein concentration for labeling is 150 $\mu$g/ml. If the concentration is lower, fractionation of the desired cell line is repeated using $1 \times 10^7$ cells/ml instead of $5 \times 10^6$ cells/ml.

Cell membrane protein labeling process

Preparation of Sulfo-NHS-Biotin Stock:

A stock solution of 20 mg/ml Sulfo-NHS-Biotin in DMSO is prepared and stored in small aliquots, avoiding refreezing once thawed. This stock is stable at -20°C for several months. A fresh solution of 2 mg/ml Sulfo-NHS-Biotin is prepared by diluting the 20 mg/ml Sulfo-NHS-Biotin stock solution 1 in 10 using DMSO.

Calculating the amount of Sulfo-NHS-Biotin required for labeling

The labeling reaction is carried out at a ratio of 5 molecules of NHS-biotin per protein molecule (B/P = 5). The formula below is used to calculate the amount of NHS-Biotin required:

$$V = P \times 1.71$$

$$V = \text{Volume in } \mu\text{l of 2mg/ml biotin required for labeling}$$

$$P = \text{membrane fraction protein concentration in mg/ml.}$$

i.e., when the membrane fraction protein concentration = 2.3 mg/ml the volume of biotin required for labeling is 2.3 x 1.71 = 3.9 μl. The average labeling ratio obtained is 3 molecules of biotin per protein molecule.

Note: NHS-Biotin should be dissolved in DMSO. The total volume of NHS-Biotin added to the labeling reaction should not exceed 10% of the labeling reaction volume. If the volume of 2 mg/ml NHS-Biotin is lower than 1 μl, the NHS-Biotin stock is diluted (in DMSO) and the calculations adjusted accordingly.

Performing the labeling

The required volume of NHS-Biotin (as calculated above) is added to 100 μl of membrane protein fraction. The labeling reaction is incubated for 2 hours at 4°C on an end-over-end shaker. The reaction is then quenched by adding 5 μl of 1M Tris-Cl, pH 7 and incubating for 15 min at 4°C. Labeled membrane protein fraction can be stored at - 20°C for up to 3 months. For longer periods, the fraction is stored at -70°C. After thawing, the sample is centrifuged at 6,000rpm rpm for 15 seconds. Detergents are removed from biotin labeled samples according to the Detergent removal procedure, described below.

**Protocol: Detergent removal**

Detergents and other additives must be removed from Glycoanalysis samples as they interfere with lectin activity. Detergent removal is performed with the "Detergent-OUT™" spin columns.

Detergent out Procedure

The column is prepared inverting several times to resuspend the resin. The bottom tip of the column is removed, and the liquid drained off. About 500 μl of equilibration buffer is applied to the column and the buffer allowed to drain off. This process is repeated 3 times.

Note: The columns are not equilibrated with organic- or Tris-based buffers.

The column is placed in a 2 ml centrifuge collection tube, centrifuged at 1000 x g for 20 to 30 seconds at room temperature, and the liquid collected in the centrifuge tube is discarded. The column is closed with a column cap, and the column placed back into the centrifuge collection tube. 200-500μl of protein solution is carefully applied to the column. After allowing to stand for 5 min, the cap is removed, the column returned to the collection tube, and centrifuged at 1000 x g for 20 to 30 seconds at room temperature. The detergent-free protein solution is collected.

The fraction concentration is determined using the Micro BCA™ Protein Assay kit (Pierce, cat. no.23235) according to manufacturers' instructions, using 15μL of sample (after detergent removal) for protein determination. Pre-dilution of sample is not required for concentration determination as a low yield is typically obtained from cell membrane fractions.

**Protocol: Glycoanalysis Using Qglycome Profiling Lectin Slides**

Protein Sample Requirements

The Qglycome Cell profiling Kit detects exposed glycans on the surface of membrane glycoproteins. The sample comprises, biotin-labeled cell-surface or total membrane glycoprotein extracts from which detergents were removed. The concentration of the analyzed protein fraction of interest on the slide must be 5-10 μg/ml. Experiments are comparative, therefore the same sample concentration must be applied to all slides.

Cy3 labeled streptavidin

Cy3 labeled streptavidin should be protected from light. All tubes or vessels containing this reagent should be wrapped in aluminum foil during storage, when used in the laboratory, and during incubations.

Preparing the slides for use

The slide pack is brought to room temperature before opening. The Incubation Frames are attached to the Qglycome profiling lectin Slides to form a chamber for the various incubation steps by peeling off the backing paper and pressing the frame onto the array slide. The array contains a frame of spots that contain blue dye, to aid in the correct placement of the frames. All the blue spots must be within the inner area of the frame. Note that the blue dye will wash off during the assay.

To record the sample ID on the slide, writing is done only on the bottom of the slide (clear area without membrane pad) using a permanent Black marker (e.g., Staedtler Black Art No. 318-9). Other permanent inks markers may stain the membrane during the incubation process.

Incubating slides

Each slide is processed in a separate 9 cm diameter Petri dish to eliminate cross-contamination. When duplicate slides are processed, both duplicates can be placed together in a 15 cm Petri dish. The volume of complete wash and block solutions required for the relevant step depends on the size of the Petri dish used. 25 ml complete wash or blocking solution is used if using a 9 cm Petri dish (single slide) or 60 ml if using a 15 cm Petri dish (duplicate slides).

All incubations and wash steps are carried out on a horizontal orbital shaker at 50 rpm. The sample or probe must be evenly distributed over the entire surface of the slide to obtain correct and reproducible results. This must be checked visually after pipetting the sample before and during the incubation.

During incubation, it is important to ensure that the membrane area of the slide is completely covered with sample or probe. The slide membrane must not dry out during incubation. The Petri dish is kept closed at all times to minimize evaporation. In order to avoid slide drying, slides are kept in the wash solution at the end of each wash step. One slide at a time is removed from the wash solution and probed with the appropriate material (sample/probe.) Once the membrane dries out, it is very hard to probe it with 450 $\mu$l of solution and the probing solution will not spread evenly on the slide. Complete wash and block solutions are not poured directly on the slide.

Preparation of reagents

Before use, reagents are prepared according the following procedures. It is important that filtered, reverse osmosis (RO) grade water is used to produce all solutions used with the Qglyco profiling Kit. This water must conform to the following specifications:

Resistivity >18 M$\Omega$ (conductivity $\mu$s/cm= 1/resistivity M$\Omega$).
Total organic carbon <5 ppb
Alternatively, HPLC-grade water can be used.

Preparing complete wash solution

All components are brought to room temperature (15-25°C) before preparing complete wash solution, ensuring that any precipitates in solutions are completely dissolved before starting.

Complete wash solution is made up by adding the reagents as specified in Table 1. At each step, it is important to ensure that the solution is stirred until any precipitate formed is dissolved before the next reagent is added.

Table 1. Components of Complete Wash Solution

| Reagent | Supplied in kit? | 500 ml | 1 liter | 2 liters |
|---|---|---|---|---|
| Buffer A 20x | Yes | 25 ml | 50 ml | 100 ml |
| Water (filtered and RO-or HPLC-grade) | No | 474 ml | 948 ml | 1896 ml |
| Solution S | Yes | 1 ml | 2 ml | 4 ml |
| Solution T* | Yes | 125 $\mu$l | 250 $\mu$l | 500 $\mu$l |
| * Filter solution with a 0.22 $\mu$M filter before adding Solution T. Stir gently after addition. | | | | |

The required amount of Buffer A x20 is diluted by adding half the required volume of R.O. water, and stirred until the solution is homogenous. The required volume of Solution S is added to the remaining R.O. water, and stirred until the solution is homogenous. The diluted Solution S is added slowly to the diluted Buffer A, while stirring, then filtered through a 0.22$\mu$m filter. The required volume of Solution T is added and stirred gently.

Complete wash solution should be stored at 2-8°C and used within 24 h, bringing to room temperature before usage.

Preparing complete blocking solution

All components are brought to room temperature (15-25°C) before preparing complete blocking solution. It is important to ensure that any precipitates in solutions are completely dissolved before starting.

Complete blocking solution is prepared according to Table 2 as described below. At each step, it is important to ensure that the solution is stirred until any precipitate formed is dissolved before the next reagent is added. Complete blocking solution is prepared freshly before each analysis.

Table 2. Components of Complete Blocking Solution

| Reagent | Supplied in kit? | For 60 ml |
|---|---|---|
| Buffer A 20x | Yes | 3 ml |
| Water (filtered and RO- or HPLC-grade) | No | 51 ml |
| Buffer B10x | Yes | 6 ml |
| Solution S | Yes | 120 μl |
| Solution T | Yes | 15 μl |

The required amount of Buffer A X20 is diluted by adding half the required volume of R.O. water, and stirring until the solution is homogenous. The required volume of Buffer B X10 is added to the diluted buffer A, and stirred until the solution is homogenous. The required volume of Solution S is added to the remaining volume of R.O. water, and stirred until the solution is homogenous. Diluted Solution S is slowly added to the diluted Buffer B while stirring. The solution is completed to final volume with R.O. if necessary. The solution is filtered through a 0.22 μm filter, the required volume of Solution T is added, and stir gently.

Preparing the labeled fraction sample for glycoanalysis

A total of 450 μl of a 5 μg/ml protein sample solution is required for each slide (2.25μg). This solution must contain 22.5 μl of complete blocking solution (see Table 3 below).

Table 3. Preparation of a Protein Sample for One Slide

| After thawing, the labeled membrane fractions are centrifuged at 6,000rpm for 15 seconds before preparing the samples for glycoanalysis. | |
|---|---|
| Component | Volume |
| Biotin-labeled cell membrane extract | 2.25μg |
| Complete blocking solution | 22.5 μl |
| Complete wash solution | to final volume of 450 μl |

Preparing Cy3-labeled streptavidin

The optimal working concentration for Cy3 labeled streptavidin has been determined to be 5 μg/ml. Cy3-labeled streptavidin was prepared as described in Table 4 below.

Table 4. Preparation of Cy3-Labeled Streptavidin for One Slide

| Component | Volume |
|---|---|
| Cy3 labeled streptavidin | 2.25μg |
| Complete blocking solution | 22.5 μl |
| Complete wash solution | to final volume of 450 μl |

**Protocol: Glycoanalysis**

Slides are processed in 9cm petri dishes. The minimal experiment requires one reference and one sample slide. The required volumes for the amount of slides processed in the experiment are prepared.

Procedure

An Incubation Frame is adhered onto each Qglycome profiling array that will be processed. The Incubation Frame must be flush with edges of the slide. Qglycome profiling lectin arrays are handled carefully, wearing non-powdered gloves during slide handling and avoiding any contact with the membrane-covered surface.

The slide(s) are placed membrane side up in a 9 cm Petri dish. 25 ml complete blocking solution is added to the Petri dish, which is then incubated on an orbital shaker set to rotate at 50 rpm for 60 min at room temperature (15-25°C). Blocking solution is discarded.

Arrays are washed by adding 25 ml complete wash solution to the Petri dish, incubating on an orbital shaker set to rotate at 50 rpm for 5 min at room temperature (15-25°C), and discarding wash solution. The wash step is repeated twice more. After the third wash step, the arrays are left submerged in wash solution to prevent them drying out.

A single array is then taken from the Petri dish and wash solution removed by pressing a paper towel to the back and edges of the array, taking care not to touch the membrane. The array is placed in a clean Petri dish and a 450 μl biotinylated protein sample is pipetted onto the membrane, ensuring that the membrane is fully covered, without touching the membrane, and avoiding formation of bubbles on the membrane. The procedure is repeated for the remaining arrays.

Arrays are incubated in the dark on an orbital shaker set to rotate at 50 rpm for 60 min at room temperature (15-25°C). During incubation, it is important to ensure that the membrane area of the arrays is completely covered with sample. The array membrane must not dry out during incubation. The lids of Petri dishes are kept on at all times to minimize evaporation. Petri dishes are covered with aluminum foil to exclude light.

Arrays are washed by adding 25 ml complete wash solution to the Petri dish, and incubated on an orbital shaker set to rotate at 50 rpm for 5 min at room temperature (15-25°C). Wash solution is discarded. The wash step is repeated twice more. After the third wash-step incubation, all arrays are kept submerged in wash solution to prevent the membranes drying out.

A single array is removed from the Petri dish and wash solution removed by pressing a paper towel to the back and edges of the array. The array is placed in a clean Petri dish and 450 μl of fluorescently CY3 labeled streptavidin (5 μg/ml) pipetted onto the membrane. The lid of the Petri dish is closed. Care is taken not to touch the membrane, and to ensure that the membrane is fully covered. Formation of bubbles on the membrane is avoided. The process is repeated for the remaining arrays.

Arrays are incubated in the dark on an orbital shaker set to rotate at 50 rpm for 20 min at room temperature (15-25°C). During incubation, care is taken to ensure that the membrane area of the arrays is completely covered with sample. The array membrane must not dry out during incubation. The lids of Petri dishes are kept on at all times to minimize evaporation. Petri dishes are covered with aluminum foil to exclude light.

Arrays are washed in the dark by adding 25 ml complete wash solution to the Petri dish, placing on an orbital shaker set to rotate at 50 rpm for 5 min at room temperature (15-25°C), and discarding wash solution. The wash procedure is repeated twice more. After the third wash step, the incubation frame is carefully peeled from each array. The arrays are washed in the dark for 1 min with 25 ml RO- or HPLC-grade water, and dried. The arrays are scanned and analyzed.

**Protocol: Drying Slides After Processing**

To avoid nonspecific background signals, slides must be dried before scanning using one of the protocols below.

Using a centrifuge:

Slide(s) are removed from final water wash, and the back of the slide(s) wiped gently with a laboratory wipe. The slides are centrifuged at 200 x g for 5-10 min (or until slides are dry) in a Coplin jar or a centrifuge slide carrier, then air dried in the dark until membrane is completely white.

If a centrifuge is not available, slides can be air dried manually.

Manual procedure:

Slide(s) are removed from final water wash, and the back of the slide(s) wiped gently with a laboratory wipe. A laboratory wipe is pressed to the sides of the membrane, taking care not to touch the central region of the membrane.

Slides are air dried in the dark until membrane is completely white.

Note: Do not cover the slides, as the condensation will re-wet the slides.

**Processed Slides storage**

Processed slides can be stored at 2-8°C up to 2 weeks in the dark. Bring slides to room temperature before scanning while keeping them in the dark.

**Scanning Slides**

Following sample processing and drying, slides should be scanned using a microarray scanner with adjustable laser power and photomultiplier tube (PMT).

EXAMPLE 2

CHARACTERIZING CELL POPULATIONS

This Example relates to the characterization of cell populations through determining glycosylation patterns or fingerprints, herein for the comparison of differentiated cells to their undifferentiated progenitor stem cells. The methods described herein may optionally be used to compare any cell populations, including cells before and after exposure to certain treatments and so forth.

The cells used in this Example may optionally be mouse embryonic stem cells (MES), which can be differentiated to neural cells as described below. The glycosylation pattern or "glycoprofile" of differentiated neural cells are compared to that of MES cells, which are not differentiated.

Materials and methods

Embryonic stem cells are usually grown on a layer of mitotically inactivated mouse primary embryonic fibroblasts to promote growth and prevent differentiation. MES cells are optionally initially grown on these fibroblasts. Alternatively or as an additional step, such MES cells may be cultured under feeder-free conditions in medium supplemented with 10% fetal calf serum and 100 U/ml recombinant leukaemia inhibitory factor (LIF) on gelatin-coated tissue culture plastic (Smith AG (1991) Culture and differentiation of embryonic stem cells. J Tiss Cult Meth 13: 89-94). Undifferentiated ES cells are expanded to ~80% confluence in a T75 flask, trypsinised and resuspended in N2B27 media (Ying QL, Smith AG (2003) Defined conditions for neural commitment and differentiation. Methods Enzymol 365: 327-341). A plurality of densities may optionally be used.

Culture medium is preferably changed each day, preferably while removing detached or dead cells. It has been reported that using this protocol, a majority of MES will start to undergo differentiation within 4-5 days, and complete differentiation can be observed after that (Conti et al, Niche-Independent Symmetrical Self-Renewal of a Mammalian Tissue Stem Cell, PLOS Biology, vol 3, issue 9, 2005).

The glycosylation pattern of MES cells before differentiation, during the differentiation process and after complete differentiation is measured, optionally by using the Qproteome Glycoprofiling Kit (Qiagen USA; the assay is performed as described in the kit manual according to manufacturer's instructions as described above).

Results: The results show that there is a change in the glycosylation pattern during the differentiation process and after complete differentiation, which may optionally be used to measure the state of differentiation of these cells.

EXAMPLE 3

DIFFERENTIATION OF 3T3L1 CELLS TO ADIPOCYTES

This Example relates to differentiation of 3T3L1 cells to adipocytes, and to the detection of different glycosylation patterns in the two different types of cells, in experiments which were actually performed.

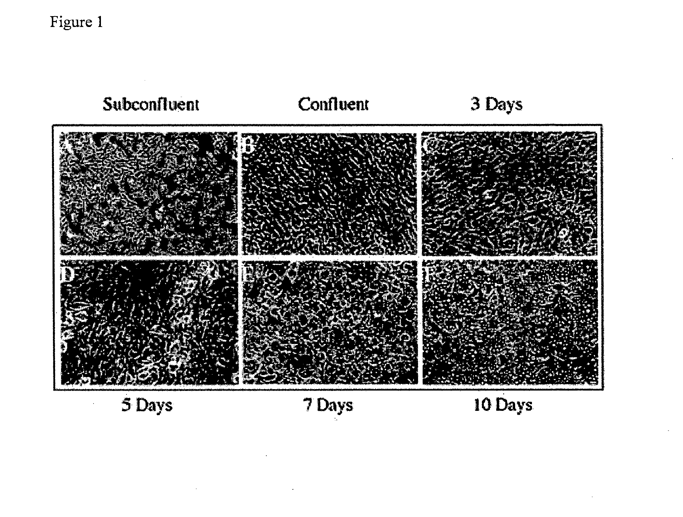

3T3L1 cells are fibroblast-like pre-adipocytes, which can be induced to differentiate to adipocytes. Adipocytes store fat, one of their hallmarks being the big fat droplets in the cytosol. During differentiation, the cells change from long, strongly adhering, fibroblast-like shaped cells to round, bright, loosely adhering cells (see Figure 1). Differentiated 3T3L1 cells represent a widely used model for lipid metabolism and insulin-dependent glucose uptake in adipocytes.

Materials and Methods: 3T3L1 cells were seeded and induced after 2 days with insulin, dexamethasone and IBMX to differentiate. 10 days after differentiation induction, when more than 80% of the cells showed large fat droplets in the cell body (see Figure 1F), cells were harvested for glycoanalysis. Control cells were maintained in regular growth medium for the same period. The cells were analyzed with the Qproteome Glycoprofiling Kit (Qiagen USA; the assay was performed as described in the kit manual according to manufacturer's instructions).

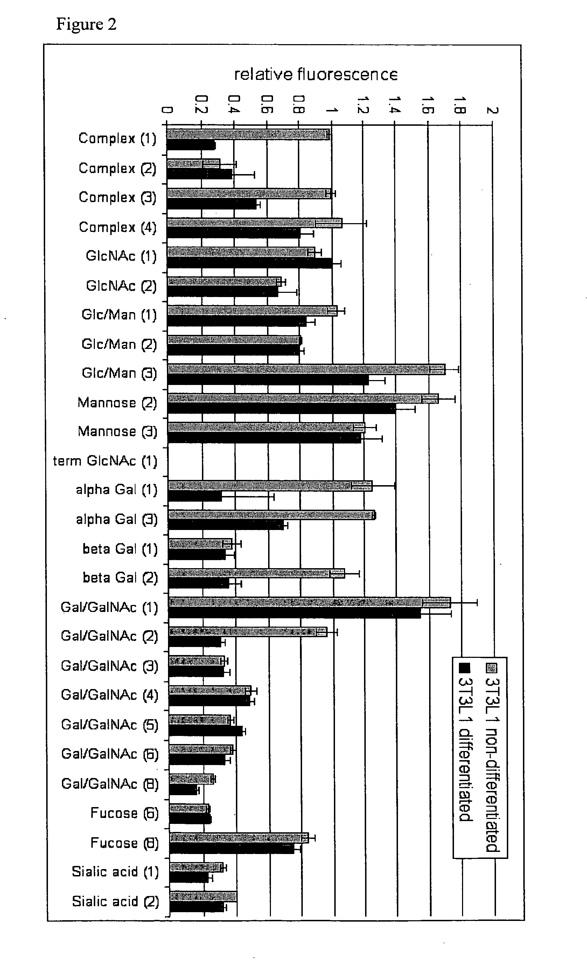

Results: As can be seen in Figure 2, the glycosylation pattern of these cells changes with differentiation. As indicated by all lectins that bind N-linked complex antennary structures and the common termini of the antennae (beta galactose and sialic acid), the antennarity is strongly reduced following differentiation. This is most pronounced in the lectin Datura stramonium, (complex(1) in Figure 2), which recognized high-antennarity glycans.

In addition, alpha-galactose is strongly reduced, as can be seen in the signal of Bandeiraea simplic (Gal Alpha (1) in Figure 2)., with concomitant reduction of high mannose structures. No significant change in O-linked glycans is observed.

This data is believed to be the first data available demonstrating global glycosylation changes during differentiation of 3T3L1 cells. The results obtained with the Qproteome Glycoprofiling Kit for determining glycosylation pattern of a cell population are consistent with previous observations that high antennary structures are involved in cell adherence, as differentiated adipocytes are only weakly adherent cells with low cell-cell interaction.

EXAMPLE 4

ER STRESS INDUCED BY BREFELDIN A

This Example relates to changes in glycosylation induced by Brefeldin A (BFA), a known inducer of endoplasmic reticulum (ER) stress, leading to apoptosis, again in experiments which were actually performed.

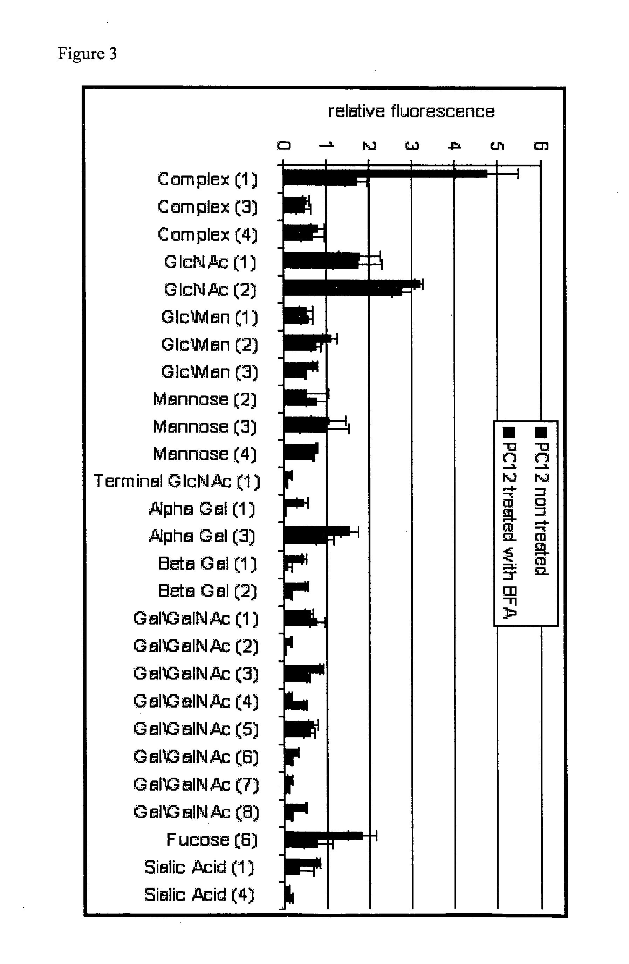

BFA inhibits protein transport from the ER to the Golgi apparatus, and has been shown to inhibit terminal glycosylation of complex N-linked glycans. BFA appears to fuse the ER and the Golgi compartments, but not the trans Golgi network (TGN). Therefore, the initial steps in the complex N-linked glycan synthesis, which occur in the cis- and medial Golgi, are inhibited only moderately. The later steps in N-glycan synthesis, like addition of the galactose, sialic acid and fucose, are performed in the TGN, and are therefore significantly inhibited by BFA (Sampath et al. (1992) J.Biol.Chem. 267, 4440-4455). PC12 cells were treated with BFA and analyzed on the lectin arrays (Figure 3).

Materials and Methods: PC12 cells were treated with 12ug/ml BFA for 24h. At this point morphological changes were observed and cells were detached. Cells were then harvested and analyzed with the Qproteome Glycoprofiling Kit (Qiagen USA; the assay was performed as described in the kit manual according to manufacturer's instructions).

Results: Following BFA treatment a significant decrease was detected in the antennarity of the membrane glycoproteins, as indicated by lectin that binds N-linked complex antennary structures (Figure 3). This is most pronounced in the lectin Datura stramonium, which recognized high antennarity glycans. Decreases in signals from lectins recognizing terminal sugars such as alpha-galactose, beta-galactose, sialic acid and fucose are also evident. This can be seen for example in the lectin Ulex europaeus I (Fucose(6) in Figure 3) which recognizes antennary fucose.

EXAMPLE 5

INHIBITION OF N-GLYCAN SYNTHESIS

This Example relates to changes in glycosylation induced by 1-deoxymannojirimycin (DMJ), a known inhibitor of mannosidase I, again in experiments that were actually performed.

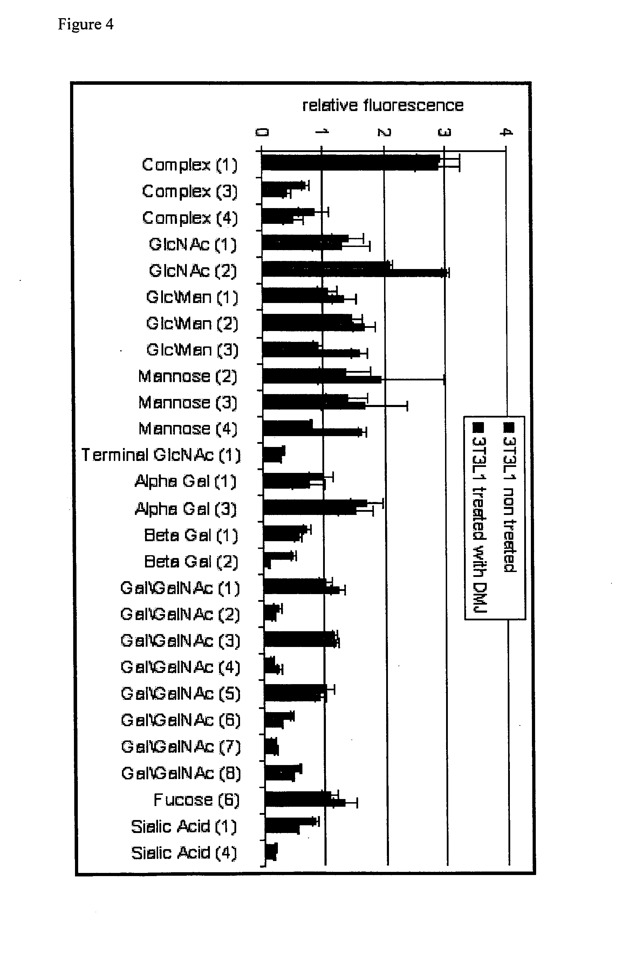

The initial steps in N-glycan synthesis involves synthesis of a precursor oligosaccharide, which is then stepwise processed by several enzymes, including mannosidase I, to allow synthesis of complex N-linked glycans. DMJ blocks mannosidase I and therefore inhibits conversion of high mannose to complex chains. As a result, treatment with DMJ leads to synthesis of glycoproteins with increased levels of high mannose glycans and less complex N-linked glycans. 3T3L1 cells were treated with DMJ; treated and untreated cells were analyzed on the lectin arrays (Figure 4).

Materials and Methods: Pre-confluent 3T3L1 cells were incubated for 3 days in the presence or absence of 800ug/ml DMJ. Cells were then harvested and analyzed with the Qproteome Glycoprofiling Kit (Qiagen USA; the assay was performed as described in the kit manual according to manufacturer's instructions).

Results: As indicated by lectins that bind N-linked complex antennary structures the antennarity is strongly reduced following DMJ treatment. Moreover, signals from all oligomannose binding lectins were significantly increased, suggesting the glycoproteins contain increased amounts of high mannose glycans in comparison to the non-treated cells. This is most pronounced in the lectins Concanavalin A (Glc/Man(3) in figure 4)) and Hippeeastrum hybrid (mannose (3) in Figure 4) which recognize mannose.

As expected (Figure 4), the obtained results show that the decrease in antennarity is also accompanied by decrease in sugars that are found on antennae termini such as beta galactose and sialic acid.

EXAMPLE 6

DIFFERENTIATION OF ADULT STEM CELLS

This Example relates to differentiation of stem cells, and to the detection of different glycosylation patterns in the differentiated and undifferentiated cells.

An adult stem cell is an undifferentiated cell found among differentiated cells in a tissue or organ, can renew itself, and can differentiate to yield the major specialized cell types of the tissue or organ. The primary roles of adult stem cells in a living organism are to maintain and repair the tissue in which they are found.

Materials and Methods:

Primary cultures of mesenchymal stem cells (MSCs) are established from normal iliac crest bone marrow aspirates from male and female donors of various ages. Culture-adherent cells are expanded, subcultured, and then tested for bone and cartilage differentiation by glycoanalysis. For a description of the culturing method see for example Haynesworth SE et al, Characterization of cells with osteogenic potential from human marrow; Bone. 1992;13(1):81-8. For a description of the method for causing such cells to differentiate, see also for example Jaiswal N et al, Osteogenic differentiation of purified, culture-expanded human mesenchymal stem cells in vitro; J Cell Biochem. 1997 Feb; 64(2): 295-312.

Control cells are maintained in regular growth medium for the same period. The cells are analyzed with the Qproteome Glycoprofiling Kit (Qiagen USA; the assay was performed as described in the kit manual according to manufacturer's instructions.

Results: As will be shown, the glycosylation pattern of these cells changes with differentiation.

EXAMPLE 7

DETERMINATION OF MALIGNANCY OF CANCER CELLS

This Example relates to determination of malignancy of cancer cells, and to the detection of different glycosylation patterns in malignant and benign types of cells, and in malignant and normal (non-cancerous) cells.

Cancer is a disease characterized by disorderly division of cells, combined with the malignant behavior of these cells. Malignant cancer cells tend to spread, either by direct growth into adjacent tissue through invasion, or by implantation into distant sites by metastasis (the process whereby cancer cells can move through the bloodstream or lymphatic system to distant locations). Benign tumors are similar to cancers in that they are composed of genetically abnormal cells which grow in excess of any normal process. However, the growth of benign tumors is self-limiting, and they do not invade other tissues or metastasize.

Materials and Methods:

Cells are isolated from a malignant tumor in a human organ, and grown in complete medium. When cells are 70-80% confluent, cells are harvested for glycoanalysis. Control cells are non-tumor cells removed from the same organ and maintained in the same growth medium for the same period. The cells are analyzed with the Qproteome Glycoprofiling Kit (Qiagen USA; the assay was performed as described in the kit manual according to manufacturer's instructions).

Results: As will be shown, the malignant and benign tumor cells show different glycosylation patterns.

As an alternative or additional method for diagnosing malignancy and/or detecting cancer, immunohistochemistry (IHC) may optionally be performed. IHC is the study of distribution of an antigen of choice in a sample based on specific binding of one or more saccharide-binding agents to whole cells, typically on tissue slices. The saccharide-binding agent features a label which can be detected, for example as a stain which is detectable under a microscope. The tissue slices are prepared by being fixed as described above. IHC is therefore particularly suitable for saccharide binding reactions that are not disturbed or destroyed by the process of fixing the tissue slices. IHC permits determining the localization of binding, and hence mapping of the presence of the saccharide within the tissue and even within different compartments in the cell. Such mapping can provide useful diagnostic information, including but not limited to the histological type of the tissue sample; the presence of specific cell types within the sample; information on the physiological and/or pathological state of cells (e.g. which phase of the cell-cycle they are in); the presence of disease related changes within the sample; differentiation between different specific disease subtypes where it is already known the tissue is of disease state (for example, the differentiation between different tumor types when it is already known the sample was taken from cancerous tissue). IHC information is valuable for more than diagnosis. It can also be used to determine prognosis and therapy treatment and monitor disease.

IHC glycoprotein markers and/or polysaccharide markers could be from any cellular location. Most often these markers are membrane proteins but secreted proteins or intracellular proteins (including intranuclear) can be used as an IHC marker too.

EXAMPLE 8

PREDICTION OF A RESPONSE OF A PATIENT TO CHEMOTHERAPY

This Example relates to prediction of a response to a patient to chemotherapy by measuring the glycolysation pattern of a cell sample before and after the therapy.

Materials and Methods:

Pre-confluent mouse 3T3L1 cells are incubated for 3 days in the presence of cisplatin, then cells are harvested for glycoanalysis by trypsinizing. Control cells are maintained in regular growth medium for the same period. Whole cell extracts are prepared by trypsinizing and resuspending as described using CE1 and CE2 extraction buffers. The cells are analyzed with the Qproteome Glycoprofiling Kit (Qiagen USA; the assay is performed as described in the kit manual according to manufacturer's instructions).

Optionally for any of the above examples, the cells may be analyzed according to methods of the present invention after being fixed.

EXAMPLE 9

METHOD FOR GLYCOANALYSIS

According to some embodiments of the present invention, the results of one or more assays with saccharide binding agents are examined according to a method for glycoanalysis, which is optionally and preferably provided in the form of software (although it may alternatively may be provided as firmware or hardware), described herein as a "comparative interpretation module". The comparative interpretation module is aimed at inferring changes in glycosylation between two samples based on significant lectin differences.

The module preferably comprises two sub-modules: a comparison module and an interpretation module. The comparison module normalizes the fingerprints and extracts the differences between them; the comparison module analyzes the list of differences in saccharide binding agent signals and deconvolutes them to provide differences in glycan epitopes. For the purpose of description only and without wishing to be limited, the method is described herein with regard to the binding behavior of lectins.

The algorithm used in this module preferably features at least one and more preferably a plurality of statistical classifiers, which have been extracted from a wide dataset of standards using machine-learning techniques. Each classifier maps a subset of lectin difference values onto a defined change in a single glycosylation epitope. The classifiers determine whether a change in a given epitope was detected, and if so, label it as an increase, decrease or (for some of the epitopes) a pattern change. Since the analyzed epitopes usually represent composite glycan structures, while the specificities of lectins are towards mono- or di-saccharides, the classifiers are based on deconvolution of signals from several lectins with overlapping and/or complementary specificities.

**Fingerprint comparison sub-module**

According to some embodiments, there is provided a fingerprint comparison sub-module. The input to the comparison sub-module is a pair of fingerprints, a reference and a target fingerprint. Initially, the fingerprints are normalized to enable a comparison of signals between the target and reference fingerprints. Following this normalization the fingerprints are compared and a list of differences is extracted.

Normalization is performed using a robust regression algorithm (the particular algorithm chosen is based on MM estimates. This algorithm extracts the largest subset of points, from both fingerprints, that produce the best possible fit. The algorithm provides both the best linear fit between the fingerprints, and an estimate of the similarity between the fingerprints, which comes from the quality of the fit. Also, the robust regression identifies the points that are outliers to the linear fit (outside the subset of the best fit), which correspond to the lectins that show appreciable changes between the fingerprints. These changes are quantified and transferred to the interpretation module.

In more details, standard regression models tend to break down when outliers exist. Robust regression methods attempt to find a fit that is independent of the existence of such outliers, by fitting a majority of the data. Regression with M estimates is an efficient, iterative method for removing outliers, provided that there are no leverage points (outliers at the extreme of the x-scale). Such leverage points cause the breakdown of the algorithm. In order to avoid this MM estimates are used. In this algorithm high-breakdown points are used to estimate the initial best-fit parameters, which are then improved iteratively by a minimization process, which in this case is optionally a Newton minimization (for example).

Interpretation classifiers

According to some embodiments, there is provided one or more interpretation classifiers. The interpretation classifiers are mathematical functions that integrate various conditions for multiple lectin differences into boolean logical terms. In cases where a single lectin signal provides a reliable signal with a clear specificity, there is a single condition based on the difference level observed for this lectin that defines epitope changes. For other cases there may be several alternative criteria, each of which if met defines a change. In this way several different combinations of changes in fingerprint can lead to the same final verdict, which is in accordance with the fact that various changes can be manifested by a different lectin sets.

**Extraction and calibration of classifier**

The above modules were tested with a benchmark of 878 fingerprint pairs that were successfully normalized in the fingerprint comparison module. These pairs were generated from 213 fingerprints from various cell lines, various biological systems, and enzymatically treated samples in which glycosylation patterns were altered in a controlled manner. Only pairs that were biologically comparable were considered for the normalization. For each pair, the expected result of at least one epitope was defined according to either (1) the particular treatment performed, (2) HPLC analyses, (3) ELISA experiments for fucose epitopes, or (4) literature reports. The benchmark was divided into nine partially overlapping training sets, each containing only pairs with a known change in a particular epitope. For each of these nine sets a set of control pairs, fingerprint pairs in which the examined change is expected not to occur, was compiled.

For each epitope, a logical rule was determined that best separates the dataset and its concomitant control. A statistical procedure was used to rank different Boolean functions that use different combinations of lectin differences, according to their ability separate the two sets. The procedure involved defining, for each epitope, a target function encompassing the sensitivity and specificity results that were obtained, and optimizing this target function on the dataset of fingerprint pairs described above. For each lectin, the minimal signal difference that is considered significant was automatically calibrated to achieve the partitioning of the highest quality. The automatically extracted rules were fine-tuned by careful manual analysis, based on the known specificities of the printed lectins. This analysis resulted in various heuristic rules that either enhance performance or deal with contradicting evidence.

Examples of calibrated rules and their concomitant verdict are listed in Table 5:

Table 5: Examples of comparative interpretation rules

| Rule* | Verdict |
|---|---|
| (d(Alpha Gal(1))>4 and d(Alpha Gal(3))≥0) or (d(Alpha Gal(1))>4 and ((d(Beta Gal (2))<0 and d(Gal\GalNAc (8))≤-1) or (d(Beta Gal (2)) ≤-1 and d(Gal\GalNAc (8))<0) or (d(Beta Gal (2)) ≤-6 and d(Beta Gal (1))<0)) or (d(Alpha Gal(3)>5) and d(Alpha Gal(1))≥0)) | Increase in α-Gal |
| d(sialic acid lectin; for example Sambucus nigra lectin)>2 and d(gal beta lectin; for example Ricinus communis agglutinin 1)<-3 | Increase in sialic acid |
| *d(XXX) = difference in lectin XXX as measured by comparison of test sample to reference sample. | |

**Results**

The performance was calculated for each epitope independently using the appropriate dataset, in which the expected interpretation of this epitope is known. The datasets are divided into a set of fingerprint pairs that are expected to show a change in the examined epitope, and a control set, in which the examined change is expected not to occur. Table 6 summarizes the performance of the algorithms on the entire benchmark. The sensitivity errors are broken down into 1-level and 2-level errors, denoting if the change was not detected (1-level), or detected in the wrong direction (2-levels). This breakdown is not applicable to the specificity analysis, since false positive detection of changes can only be a 1-level error.

**Table 6: performance summary**

| | | Sensitivity analysis | | | Specificity analysis |
|---|---|---|---|---|---|
| | | Sensitivity | 1 level error | 2 level error | Specificity |
| N-linked glycans | | | | | |
| | antennarity of complex glycans | 71% | 29% | 0% | 89% |
| | oligomannose epitopes | 68% | 32% | 0% | 89% |
| O-linked glycans | | | | | |
| | global pattern change | 50% | NA* | NA | 72% |
| **Terminal sugars** | | | | | |

(continued)

| | | Sensitivity analysis | | | Specificity analysis |
| --- | --- | --- | --- | --- | --- |
| | | Sensitivity | 1 level error | 2 level error | Specificity |
| | sialic acid | 74% | 26% | 0% | 81% |
| | beta-galactose | 79% | 21% | 0% | 88% |
| | alpha-galactose | 79% | 18% | 2% | 80% |
| | GlcNAc | 100% | 0% | 0% | 95% |
| | fucose-alpha(1-2) | 74% | 23% | 3% | 95% |
| | fucose (other) | 54% | 45% | 1% | 96% |
| * Since the function for O-linked glycans identifies a change in global pattern, the breakdown of errors is not applicable. | | | | | |

These results clearly indicate that the method and modules described above are sufficient to be accurate discriminators between different types of binding results.

While the invention has been described with respect to a limited number of embodiments, it will be appreciated that many variations, modifications and other applications of the invention may be made.

**Claims**

1.  An in vitro method for detecting a state of malignancy of_a cell, comprising:

    contacting at least a portion of the cell with at least one saccharide binding agent; determining binding of said saccharide binding agent to said cell;
    determining said glycosylation pattern according to binding of said at least one saccharide binding agent; and correlating said glycosylation pattern to the state of the cell.

2.  The method of claim 1, wherein said portion of said cell is a cell preparation which is selected from the group consisting of a membrane protein extract, a homogenized cell, and a crude membrane mixture.

3.  The method of claim 1 or 2 wherein said contacting said at least a portion of the cell comprises:

    providing on a surface of a substrate a plurality of different essentially sequence- and/or site-specific saccharide-binding agents, which bind saccharide-recognition sequences of a polysaccharide, wherein a number of the plurality of said different essentially sequence- and/or site-specific saccharide binding agents are immobilized on the same surface of said substrate;
    contacting said surface with a polysaccharide to be analyzed, or with a mixture comprising a plurality of fragments of said polysaccharide, of the cell;
    washing or otherwise removing unbound polysaccharide or polysaccharide fragments;
    adding to the obtained surface an essentially sequence- and/or site-specific saccharide-binding marker, or a mixture of essentially sequence- and/or site-specific saccharide-binding markers, wherein said marker or mixture of markers binds said bound polysaccharide; and
    detecting binding of said saccharide-binding markers that are bound to said surface.

4.  The method of claim 3, wherein said detection binding of said saccharide-binding markers comprises visual inspection, or wherein said detecting binding of said saccharide-binding markers comprises:

    acquiring one or more images of said bound saccharide-binding markers; and
    generating from said one or more images, a map of recognition sites of said polysaccharide being analyzed, thereby deriving partial sequence information of said polysaccharide.

5.  The method of claim 4, wherein said markers are chromogenic binding agents, and wherein said images of said markers are colors that develop on said surface or wherein said markers are labeled binding agents, and wherein said images of said markers are provided according to a signal from said label.

6. The method of claim 4, wherein said acquiring said one or more images comprises the use of optical filters.

7. The method of claim 4, wherein said acquiring said one or more images comprises photographing and/or digitizing said images.

8. The method of claim 1, wherein said essentially sequence- and/or site- specific binding agents are lectins or antibodies.

9. The method of claim 8, wherein said lectins or antibodies_are colored lectins/antibodies, fluorescent lectins/antibodies, or biotin labelled lectins/antibodies.

10. The method of claim 8 or 9 wherein the sequence- and/or site- specific binding agents comprise at least five lectins.

11. The method of claim 1, wherein said correlating said glycosylation pattern to the state of the cell comprises comparison of said glycosylation pattern to at least one known category, preferably wherein said glycosylation pattern is computationally analyzed.

12. The method of claim 3, wherein said surface comprises a bead or

13. The method of claim 1 for predicting a response of a patient to a therapy, preferably chemotherapy,_comprising:

    measuring a glycosylation pattern of a cell of the patient; and
    correlating said glycosylation pattern to the predicted response of the patient to therapy.

14. The method of any of claims 1 - 13, further comprising addition of detergent to said cell and optionally further comprising solubilization of cell membranes, and optionally further comprising extraction of membrane proteins.

15. A kit for performing a method according to any of claims 1 - 14.

Figure 1

Subconfluent     Confluent     3 Days

5 Days     7 Days     10 Days

Figure 2

Figure 3

relative fluorescence

Complex (1)
Complex (3)
Complex (4)
GlcNAc (1)
GlcNAc (2)
Glc\Man (1)
GlcWan (2)
GlcWan (3)
Mannose (2)
Mannose (3)
Mannose (4)
Terminal GlcNAc (1)
Alpha Gal (1)
Alpha Gal (3)
Beta Gal (1)
Beta Gal (2)
Gal\GalNAc (1)
Gal\GalNAc (2)
Gal\GalNAc (3)
Gal\GalNAc (4)
Gal\GalNAc (5)
Gal\GalNAc (6)
Gal\GalNAc (7)
Gal\GalNAc (8)
Fucose (6)
Sialic Acid (1)
Sialic Acid (4)

■ PC12 non treated
■ PC12 treated with BFA

Figure 4

## EUROPEAN SEARCH REPORT

Application Number

EP 11 16 6862

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ROSENFELD R ET AL: "U-C FINGERPRINT: GLYCOPROTEIN ANALYSIS BASED ON A LECTIN ARRAY", GLYCOBIOLOGY, IRL PRESS,, GB, vol. 13, no. 11, November 2003 (2003-11), XP008049528, ISSN: 0959-6658 * abstract * | 1-15 | INV. G01N33/50 |
| X,D | WO 02/37106 A (GLYCODATA LTD [IL]; MARKMAN OFER [IL]; ROTHMAN CHANA [IL]; AMOR YEHUDI) 10 May 2002 (2002-05-10) * pp.5-6, 14, 18-26, 32-33 * * page 4 - page 6 * * page 19 - page 27 * * page 31 - page 38 * * page 46; examples 5,6 * | 1-15 | |
| X | US 7 056 678 B1 (MARKMAN OFER [IL]) 6 June 2006 (2006-06-06) * col.4, 9, col.11 par.2examples 5,6 * * column 4, line 1 - column 6, line 13 * * column 15 - column 16 * | 1-15 | TECHNICAL FIELDS SEARCHED (IPC) G01N |
| A | MURAMATSU ET AL: "Alterations of cell-surface carbohydrates during differentiation and development", BIOCHIMIE, MASSON, PARIS, FR, vol. 70, no. 11, 1 November 1988 (1988-11-01), pages 1587-1596, XP023480375, ISSN: 0300-9084, DOI: 10.1016/0300-9084(88)90294-5 [retrieved on 1988-11-01] | 1-11 | |

-/--

The present search report has been drawn up for all claims

| Place of search | Date of completion of the search | Examiner |
|---|---|---|
| Munich | 22 November 2011 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | Application Number EP 11 16 6862 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RAEDLER A ET AL: "The use of lectins to study normal differentiation and malignant transformation", JOURNAL OF CANCER RESEARCH AND CLINICAL ONCOLOGY, SPRINGER INTERNATIONAL, BERLIN, DE, vol. 109, no. 3, 1 January 1985 (1985-01-01), pages 245-251, XP009134784, ISSN: 0171-5216 * abstract; p.248 col.1 - p.249 * | 1-15 | |
| X | KANOELANI T PILOBELLO ET AL: "Development of a Lectin Microarray for the Rapid Analysis of Protein Glycopatterns", CHEMBIOCHEM - A EUROPEAN JOURNAL OF CHEMICAL BIOLOGY, WILEY VCH, WEINHEIM, DE, vol. 6, 1 January 2005 (2005-01-01), pages 985-989, XP007913607, ISSN: 1439-4227, DOI: 10.1002/CBIC.200400403 * figures * | 1-15 | |
| X | "Qproteome GlycoArray Handbook", QPROTEOME GLYCOARRAY HANDBOOK,, 1 September 2005 (2005-09-01), pages 1-60, XP007913608, * pp.7-12 * | 1-15 | TECHNICAL FIELDS SEARCHED (IPC) |
| X | HAKOMORI: "Tumor malignancy defined by aberrant glycosylation and sphingo(glyco)lipid metabolism.", CANCER RESEARCH, vol. 56, no. 23, 1 December 1996 (1996-12-01), pages 5309-5318, XP55012222, ISSN: 0008-5472 * abstract; figures, in particular figure 1 * | 1-15 | |

The present search report has been drawn up for all claims

| Place of search | Date of completion of the search | Examiner |
|---|---|---|
| Munich | 22 November 2011 | Behrens, Ralf |

EPO FORM 1503 03.82 (P04C01)

## ANNEX TO THE EUROPEAN SEARCH REPORT
## ON EUROPEAN PATENT APPLICATION NO.

EP 11 16 6862

This annex lists the patent family members relating to the patent documents cited in the above-mentioned European search report.
The members are as contained in the European Patent Office EDP file on
The European Patent Office is in no way liable for these particulars which are merely given for the purpose of information.

22-11-2011

| Patent document cited in search report | | Publication date | Patent family member(s) | | Publication date |
|---|---|---|---|---|---|
| WO 0237106 | A | 10-05-2002 | AU | 2599202 A | 15-05-2002 |
| | | | AU | 4160402 A | 11-06-2002 |
| | | | CA | 2428150 A1 | 10-05-2002 |
| | | | CA | 2428431 A1 | 06-06-2002 |
| | | | EP | 1334359 A2 | 13-08-2003 |
| | | | EP | 1402450 A2 | 31-03-2004 |
| | | | EP | 2256653 A2 | 01-12-2010 |
| | | | IL | 155716 A | 03-08-2009 |
| | | | IL | 155717 A | 03-08-2009 |
| | | | JP | 4176467 B2 | 05-11-2008 |
| | | | JP | 4696100 B2 | 08-06-2011 |
| | | | JP | 2004533599 A | 04-11-2004 |
| | | | JP | 2008020463 A | 31-01-2008 |
| | | | JP | 2008029347 A | 14-02-2008 |
| | | | WO | 0237106 A2 | 10-05-2002 |
| | | | WO | 0244714 A2 | 06-06-2002 |
| US 7056678 | B1 | 06-06-2006 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0068688 A
- WO 0184147 A
- US 20060194269 A
- US 20070092915 A
- US 7056678 B
- US 7132251 B
- WO 0237106 A
- US 20040132131 A
- WO 0244714 A
- US 7079955 B
- US 20040153252 A
- US 20050186645 A

### Non-patent literature cited in the description

- **SANDERS et al.** A high-yield technique for preparing cells fixed in suspension for scanning electron microscopy. *The Journal of Cell Biology,* 1975, vol. 67, 476-480
- **NIMRICHTER et al.** *Intact cell adhesion to glycan microarrays, Glycobiology,* 2004, vol. 14 (2), 197-203
- **SMITH AG.** Culture and differentiation of embryonic stem cells. *J Tiss Cult Meth,* 1991, vol. 13, 89-94
- **YING QL ; SMITH AG.** Defined conditions for neural commitment and differentiation. *Methods Enzymol,* 2003, vol. 365, 327-341
- **CONTI et al.** Niche-Independent Symmetrical Self-Renewal of a Mammalian Tissue Stem Cell. *PLOS Biology,* 2005, vol. 3 (9
- **SAMPATH et al.** *J.Biol.Chem.,* 1992, vol. 267, 4440-4455
- **HAYNESWORTH SE et al.** Characterization of cells with osteogenic potential from human marrow. *Bone,* 1992, vol. 13 (1), 81-8
- **JAISWAL et al.** Osteogenic differentiation of purified, culture-expanded human mesenchymal stem cells in vitro. *J Cell Biochem.,* February 1997, vol. 64 (2), 295-312